# EUROPEAN PATENT APPLICATION

(11) **EP 3 939 576 A1**
(43) Date of publication of application: **19.01.2022**
(21) Application number: 20305807.8
(22) Date of filing: 15.07.2020
(51) Int. Cl.: A61K 31/00, A61K 9/00, A61K 31/4706, A61K 31/513, A61K 31/675, A61K 31/683, A61K 31/706, A61P 31/12, A61P 31/14, A61P 31/18

(54) **PHARMACEUTICAL COMPOSITIONS FOR TREATING INFECTIONS BY A NEUROTROPIC VIRUS**

(71) Applicant: Ceres Brain Therapeutics, 75014 Paris (FR); Commissariat à l'Energie Atomique et aux Energies Alternatives (CEA), 75015 Paris (FR)
(72) Inventor: BENECH, Henri, 75005 Paris (FR); MABONDZO, Aloïse, 75013 Paris (FR); JOUDINAUD, Thomas, 75017 Paris (FR)
(74) Representative: Vial, Lionel

(57) **Abstract**

The present invention relates to a pharmaceutical composition, comprising at least one antiviral agent as an active agent, for use in the prevention or treatment of an infection by a neurotropic virus, in particular by a virus of the Coronaviridae family, wherein the pharmaceutical composition is administered by the nasal route, in particular through the olfactory nerve pathway.

SEQUENCE LISTING

<110>  CERES BRAIN Therapeutics
       Commissariat à l'Energie Atomique et aux Energies Alternatives (CEA)

<120>  PHARMACEUTICAL COMPOSITIONS FOR TREATING INFECTIONS BY A NEUROTROPIC
VIRUS

<130>  B000118EP

<160>  1

<170>  PatentIn version 3.5

<210>  1
<211>  29903
<212>  DNA
<213>  SARS-CoV-2

<400>  1
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc     180

ttctgcaggc tgcttacggt ttcgtccgtg ttgcagccga tcatcagcac atctaggttt     240

cgtccgggtg tgaccgaaag gtaagatgga gagccttgtc cctggtttca acgagaaaac     300

acacgtccaa ctcagtttgc ctgttttaca ggttcgcgac gtgctcgtac gtggctttgg     360

agactccgtg gaggaggtct tatcagaggc acgtcaacat cttaaagatg gcacttgtgg     420

cttagtagaa gttgaaaaag gcgttttgcc tcaacttgaa cagccctatg tgttcatcaa     480

acgttcggat gctcgaactg cacctcatgg tcatgttatg gttgagctgg tagcagaact     540

cgaaggcatt cagtacggtc gtagtggtga gacacttggt gtccttgtcc ctcatgtggg     600

cgaaatacca gtggcttacc gcaaggttct tcttcgtaag aacggtaata aaggagctgg     660

tggccatagt tacggcgccg atctaaagtc atttgactta ggcgacgagc ttggcactga     720

tccttatgaa gattttcaag aaaactggaa cactaaacat agcagtggtg ttacccgtga     780

actcatgcgt gagcttaacg gaggggcata cactcgctat gtcgataaca acttctgtgg     840

ccctgatggc taccctcttg agtgcattaa agaccttcta gcacgtgctg gtaaagcttc     900

atgcactttg tccgaacaac tggactttat tgacactaag aggggtgtat actgctgccg     960

tgaacatgag catgaaattg cttggtacac ggaacgttct gaaaagagct atgaattgca    1020

gacacctttt gaaattaaat tggcaaagaa atttgacacc ttcaatgggg aatgtccaaa    1080

ttttgtattt cccttaaatt ccataatcaa gactattcaa ccaagggttg aaaagaaaaa    1140

gcttgatggc tttatgggta gaattcgatc tgtctatcca gttgcgtcac caaatgaatg    1200

caaccaaatg tgcctttcaa ctctcatgaa gtgtgatcat tgtggtgaaa cttcatggca    1260

gacgggcgat tttgttaaag ccacttgcga attttgtggc actgagaatt tgactaaaga    1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 2940

actgggcatt gatttagatg agtggagtat ggctacatac tacttatttg atgagtctgg 3000

tgagtttaaa ttggcttcac atatgtattg ttctttctac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

taaggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttgttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacctcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgtct ggttttaagc taaaagactg tgttatgtat gcatcagctg tagtgttact 11340

aatccttatg acagcaagaa ctgtgtatga tgatggtgct aggagagtgt ggacacttat 11400

gaatgtcttg acactcgttt ataaagttta ttatggtaat gctttagatc aagccatttc 11460

catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccaccta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140
gtttaagaat attgatggtt attttaaaat atattctaag cacacgccta ttaatttagt 22200
gcgtgatctc cctcagggtt tttcggcttt agaaccattg gtagatttgc caataggtat 22260
taacatcact aggtttcaaa ctttacttgc tttacataga agttatttga ctcctggtga 22320
ttcttcttca ggttggacag ctggtgctgc agcttattat gtgggttatc ttcaacctag 22380
gacttttcta ttaaaatata atgaaaatgg aaccattaca gatgctgtag actgtgcact 22440
tgaccctctc tcagaaacaa agtgtacgtt gaaatccttc actgtagaaa aaggaatcta 22500
tcaaacttct aactttagag tccaaccaac agaatctatt gttagatttc ctaatattac 22560
aaacttgtgc ccttttggtg aagtttttaa cgccaccaga tttgcatctg tttatgcttg 22620
gaacaggaag agaatcagca actgtgttgc tgattattct gtcctatata attccgcatc 22680
attttccact tttaagtgtt atggagtgtc tcctactaaa ttaaatgatc tctgctttac 22740
taatgtctat gcagattcat ttgtaattag aggtgatgaa gtcagacaaa tcgctccagg 22800
gcaaactgga aagattgctg attataatta taaattacca gatgatttta caggctgcgt 22860
tatagcttgg aattctaaca atcttgattc taaggttggt ggtaattata attacctgta 22920
tagattgttt aggaagtcta atctcaaacc ttttgagaga gatatttcaa ctgaaatcta 22980
tcaggccggt agcacacctt gtaatggtgt tgaaggtttt aattgttact ttcctttaca 23040
atcatatggt ttccaaccca ctaatggtgt tggttaccaa ccatacagag tagtagtact 23100
ttcttttgaa cttctacatg caccagcaac tgtttgtgga cctaaaaagt ctactaattt 23160
ggttaaaaac aaatgtgtca atttcaactt caatggttta acaggcacag gtgttcttac 23220
tgagtctaac aaaaagtttc tgcctttcca acaatttggc agagacattg ctgacactac 23280
tgatgctgtc cgtgatccac agacacttga gattcttgac attacaccat gttcttttgg 23340
tggtgtcagt gttataacac caggaacaaa tacttctaac caggttgctg ttctttatca 23400
ggatgttaac tgcacagaag tccctgttgc tattcatgca gatcaactta ctcctacttg 23460
gcgtgtttat tctacaggtt ctaatgtttt tcaaacacgt gcaggctgtt taataggggc 23520
tgaacatgtc aacaactcat atgagtgtga catacccatt ggtgcaggta tatgcgctag 23580
ttatcagact cagactaatt ctcctcggcg ggcacgtagt gtagctagtc aatccatcat 23640
tgcctacact atgtcacttg gtgcagaaaa ttcagttgct tactctaata actctattgc 23700
catacccaca aattttacta ttagtgttac cacagaaatt ctaccagtgt ctatgaccaa 23760
gacatcagta gattgtacaa tgtacatttg tggtgattca actgaatgca gcaatctttt 23820
gttgcaatat ggcagttttt gtacacaatt aaaccgtgct ttaactggaa tagctgttga 23880
acaagacaaa aacacccaag aagtttttgc acaagtcaaa caaatttaca aaacaccacc 23940
aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag cttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acggttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagac aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaacgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta ggggaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagaatgac aaaaaaaaaa 29880

aaaaaaaaaa aaaaaaaaaa aaa 29903

## Description

### Field of the invention

The present invention relates to compositions and methods for treating infections by neurotropic viruses, in particular by viruses of the *Coronaviridae* family, more particularly by the virus SARS-CoV-2.

### Technical background

In December 2019 an outbreak of pneumonia cases of unknown origin in occurred in Wuhan in China and spread quickly nationwide. On January 7, 2020, the causative pathogen was identified as a novel coronavirus, which was named 2019-nCoV and later SARS-CoV-2.

The new virus is closely related to both SARS-CoV (82% nucleotide identity) and MERS-CoV (50% nucleotide identity), yet distinct from them.

Early mortality rates suggested that COVID-19, the name for the disease caused by SARS-CoV-2, may be less severe than SARS and MERS. However, illness onset among rapidly increasing numbers of people rapidly suggested that SARS-CoV-2 would be more contagious than both SARS-CoV and MERS-CoV. As of May 11, 2020 4 063 525 cases of COVID-19 (in accordance with the applied case definitions and testing strategies in the affected countries) have been reported, including 282 244 deaths. A great deal of effort has been made to find effective drugs against the virus. Among the various compounds tested, remdesivir, a drug previously developed for the treatment of Ebola virus infections, has been reported to show promising efficacy and acceptable safety in treating COVID-19 in a news release of the National Institutes of Health (NIH) dated April 29, 2020. As such, preliminary results indicate that patients who received remdesivir had a 31% faster time to recovery than those who received placebo (p<0.001). Specifically, the median time to recovery was 11 days for patients treated with remdesivir compared with 15 days for those who received placebo. Results also suggested a survival benefit, with a mortality rate of 8.0% for the group receiving remdesivir versus 11.6% for the placebo group (p=0.059).

However, the efficacy of remdesivir is not completely established yet as Wang et al. (2020) Lancet doi.org/10.1016/S0140-6736(20)31022-9 report that in their trial remdesivir use was not associated with a difference in time to clinical improvement.

Accordingly, there is still a need for alternative treatments of infections by SARS-CoV-2.

### Summary of the invention

The present invention follows from the unexpected finding, by the inventors, that administration of antiviral agents through the olfactory nerve route could improve their efficacy against neurotropic viruses, in particular against viruses of the *Coronaviridae* family, more particularly against SARS-CoV-2.

Without wishing to be bound to a particular theory, the inventors believe that one of the reasons antiviral drugs used against SARS-CoV-2 are ineffective lies in the fact that the virus could escape these drugs by circulating in the nervous system, in particular through infection of the olfactory nerve.

Accordingly, the present invention relates to a pharmaceutical composition, comprising at least one antiviral agent as an active agent, for use in the prevention or treatment of an infection by a neurotropic virus, in particular by a virus of the *Coronaviridae* family, wherein the pharmaceutical composition is administered by the nasal route, in particular through the olfactory nerve pathway.

### Description of the invention

As intended herein, the word "comprising" is synonymous to "include" or "contain". When a subject-matter is said to comprise one or several features, it is meant that other features than those mentioned can be comprised in the subject-matter. Conversely, the expression "constituted of" is synonymous to "consisting of". When a subject-matter is said to consist of one or several features, it is meant that no other features than those mentioned are comprised in the subject-matter.

### Virus

As intended herein the infection to be prevented or treated is due to a neurotropic virus. Neurotropic viruses, which are well known to one of skill in the art, are capable of infecting nerve cells. Neurotropic viruses according to the invention can be neuroinvasive and/or neurovirulent. Neurotropic viruses according to the invention can infect the central nervous system, in particular the brain, and/or the peripheral nervous system.

Neurotropic viruses according to the invention are preferably selected from the group consisting of Japanese Encephalitis virus, Venezuelan Equine Encephalitis virus, California encephalitis virus; polio virus, coxsackie virus, echo virus, mumps virus, measles virus, influenza virus, rabies virus, , varicella-zoster virus, Epstein-Barr virus, cytomegalovirus, HHV-6 virus, rubella virus, JC virus, a virus of the Retroviridae family, a virus of the Herpesviridae family, and a virus of the Coronaviridae family.

A preferred neurotropic virus of the Retroviridiae family according to the invention is Human Immunodeficiency Virus (HIV), in particular HIV-1 or HIV-2, or human T-lymphotropic virus 1.

A preferred neurotropic virus of the Herpesviridae family according to the invention is Herpes Simplex Virus (HSV), in particular HSV-1 or HSV-2.

Prerebaly, the infection to be prevented or treated is due to a virus of the *Coronaviridae* family.

Preferably, the virus as defined above is of the *Alphacoronavirus*, *Betacoronavirus,* Deltacoronavirus, or *Gammacoronavirus* genus, more preferably of the *Betacoronavirus* genus, most preferably of the *Sarbecovirus* or the *Merbecovirus* subgenus.

Preferably also the virus as defined above is a human virus, i.e. a virus which can infect a human.

Preferably, the virus as defined above is selected from the group consisting of SARS-CoV, SARS-CoV-2, MERS-CoV and mutants or variants thereof.

Preferably, the virus as defined above is SARS-CoV-2, or a mutant or variant thereof. SARS-CoV-2 is notably described in Fuk-Woo Chan et al. (2020) Emerging Microbes & Infections 9:221-236, which is incorporated herein by reference, and is also named 2019-nCoV, HCoV-19, SARS2, COVID-19 virus, Wuhan coronavirus, Wuhan seafood market pneumonia virus, Human coronavirus 2019.

Preferably, the virus as defined above is SARS-CoV-2 and has the genomic sequence defined by NCBI Reference Sequence NC_045512.2 (SEQ ID NO: 1), or the complementary thereof, or is a mutant or variant thereof. Mutant or variants of SEQ ID NO: 1 can in particular be found on the "NCBI virus" website by searching for SARS-CoV-2 taxid:2697049.

As intended herein, a "mutant or variant" of a virus as defined above, or of a genomic sequence of a virus as defined above, has a genomic sequence or is a nucleotide sequence which has at least 85%, 90%, 95%, 96% 97%, 98%, 99% or 99,5% identity with the genomic sequence of the virus as defined above.

As intended herein, a first nucleotide sequence "having at least X% identity" with a second nucleotide sequence, in particular differs from the second sequence by the insertion, the suppression or the substitution of at least one nucleotide. Besides, the percentage of identity between two nucleotide sequences is defined herein as the number of positions for which the bases are identical when the two sequences are optimally aligned, divided by the total number of bases of the longer of the two sequences. Two sequences are said to be optimally aligned when the percentage of identity is maximal. Besides, as will be clear to one of skill in the art, it may be necessary to add gaps in order to obtain an optimal alignment between the two sequences. In addition, when calculating the percentage of identity between an RNA nucleotide sequence and a DNA nucleotide sequence, an Uracile (U) base and a Thymine (T) base at the same position are considered to be identical.

As intended herein preventing or treating an infection by a virus of the *Coronaviridae* family in an individual, encompasses preventing or treating the symptoms, disorders, syndromes, conditions or diseases, such as pneumonia or COVID-19, associated to the infection by the virus of the *Coronaviridae* family, more particularly by SARS-CoV-2. Preferably, the pharmaceutical composition as defined above is for use for preventing or treating anosmia associated with the viral infection.

Preferably, the pharmaceutical composition as defined above is for use for preventing or treating dysgeusia associated with the viral infection.

Preferably, the pharmaceutical composition as defined above is for use for preventing or treating encephalopathy associated with the viral infection.

Preferably, the pharmaceutical composition as defined above is for use for preventing or treating neuro-inflammation associated with the viral infection.

### Individual

Preferably, the individual is a bird, such as a chicken, or a mammal, such as a human, a canine, in particular a dog, a feline, in particular a cat, an equine, a bovine, a porcine, a caprine, such a sheep or a goat, or a camelidae, more preferably the individual is a human.

Preferably, the individual as defined above is a human aged 50 or more, more preferably 60 or more, even more preferably 70 or more and most preferably 80 or more.

Preferably, the individual as defined above is a male individual.

Preferably, the individual as defined above suffers from at least one other disease or condition, in particular selected from hypertension, diabetes, in particular type 2 diabetes, metabolic syndrome, a cardiovascular disease, in particular ischemic cardiomyopathy, a chronic respiratory disease, or cancer.

Preferably, the individual as defined above is overweight or obese.

According to a usual definition a human individual is considered overweight if its Body Mass Index (BMI, body weight in kg relative to the square of the height in meters) is higher than or equal to 25 kg/m² and less than 30 kg/m² and the individual will be said obese if his BMI is higher than or equal to 30 kg/m². The individual according to the invention may notably present with severe obesity, in particular characterized in human by a BMI higher than or equal to 35 kg/m².

More generally, it is preferred that the individual as defined above is a human and has a BMI higher than or equal to 25 kg/m², 26 kg/m², 27 kg/m², 28 kg/m², 29 kg/m², 30 kg/m², 31 kg/m², 32 kg/m², 33 kg/m², 34 kg/m², 35 kg/m² or 40 kg/m².

Besides, the individual as defined above may also have an abdominal obesity, corresponding in particular to a visceral adipose tissue excess. According to a usual definition a male human individual has an abdominal obesity if the abdominal perimeter is higher than or equal to 94 cm, in particular higher than 102 cm and a female human individual has an abdominal obesity if the abdominal perimeter is higher than or equal to 80 cm, in particular higher than 88 cm. The abdominal perimeter measure is well known to one of skilled in the art: abdomen circumference is thus preferably measured midway between the last floating rib and the top of the iliac crest in a standing individual in gentle expiration.

It is particularly preferred that the individual as defined above is a man and presents with an abdominal perimeter higher than or equal to 90 cm, 91 cm, 92 cm, 93 cm, 94 cm, 95 cm, 96 cm, 97 cm, 98 cm, 99 cm, 100 cm, 101 cm or 102 cm. It is also preferred that the individual according to the invention is a woman and presents with an abdominal perimeter higher than or equal to 75 cm, 76 cm, 77 cm, 78 cm, 79 cm, 80 cm, 81 cm, 82 cm, 83 cm, 84 cm, 85 cm, 86 cm, 87 cm or 88 cm.

Preferably, the individual according to the invention is afflicted with COVID-19 or is at risk of being afflicted with COVID-19.

Preferably, the individual according to the invention is afflicted with anosmia or is at risk of being afflicted with anosmia.

Preferably, the individual according to the invention is afflicted with dysgeusia or is at risk of being afflicted with dysgeusia.

Preferably, the individual according to the invention is afflicted with encephalopathy or is at risk of being afflicted with encephalopathy.

Preferably, the individual according to the invention is afflicted with neuro-inflammation or is at risk of being afflicted with neuro-inflammation.

### Antiviral agents

As intended herein any antiviral agent which can prevent or treat an infection by a neurotropic virus, in particular by a virus of the *Coronaviridae* family, can be used according to the invention. The antiviral agent may in particular be an inhibitor of the viral polymerase (such as Nsp12 of SARS-CoV-2 or the reverse transcriptase (RT) of HIV), of the viral helicase (such as Nsp13 or SARS-CoV-2), of the viral integrase (such as HIV integrase) or of the viral protease (such HIV protease); an inhibitor of the binding of the virus to its target receptor(s) (such as ACE-2 for SARS-CoV2-2 or CD4, CCR5 or CXCR4 for HIV); a fusion inhibitor; or a general antiviral agent, such as an interferon. However it is preferred that the antiviral agent is selected from the group consisting of hydroxychloroquine; chloroquine; atovaquone; remdesivir; ribavirin; didanosine; penciclovir; favipravir; nafamostat; cobicistat; nitazoxanide; azithromycin; carrimycin; recombinant human interferon a1β; peginterferon alfa-2a; oseltamivir; nicotine; tofacitinib; imatinib; acalabrutinib; losartan; valsartan; telmisartan; tetrandine; clevudine; tiliquinone; nitazoxanide; tilbroquinone; nifurzide; nifuroxazide; minocycline; rovomycine; rocephine; HIV reverse transcriptase inhibitors, such as tenofovir; HIV protease inhibitors, such as lopinavir, ritonavir, lopinavir/ritonavir, darunavir, danoprevir, umifenovir; purine analogues; and pharmaceutically acceptable salts, derivatives and prodrugs thereof.

More preferably, the antiviral agent is selected from the group consisting of remdesivir, hydroxychloroquine, tenofovir and lopinavir.

### Pharmaceutical composition

Preferably, the pharmaceutical composition as defined above further comprises at least one pharmaceutically acceptable vehicle, carrier or excipient suitable for an administration through the nasal route, in particular through the olfactory nerve pathway.

Vehicles, carriers or excipients suitable for an administration through the nasal route are well known to one of skill in the art and may in particular be selected from the group consisting of a fatty or a lipophilic solvent or phase and an aqueous or hydrophilic solvent or phase.

The fatty or lipophilic solvent or phase may comprise or consist of at least one fatty acid, monoglyceride, diglyceride, triglyceride, fatty alcohol, fatty ester or combinations thereof.

By way of example, the fatty acid may in particular be docosahexaenoic acid or isopropyl palmitate.

By way of example the triglyceride may in particular be Caprylic/Capric Triglyceride. Maisine^{®} CC (Gattefosse), also named Glycerol/Glyceryl monolinoleate, is an example of a combination of mono-, di-, and triglycerides.

By way of example, the fatty alcohol may in particular be myristat isopropyl alcohol or stearyl alcohol.

By way of example the fatty ester may in particular be isopropyl lauroyl sarcosinate. The aqueous or hydrophilic solvent or phase may comprise or consist of water, a glycol, such as glycerol, carboxymethyl cellulose, benzyl alcohol or combinations thereof.

The aqueous an aqueous or hydrophilic solvent or phase may also comprise with pH-modifiers such as citric acid or hydrochloric acid.

The aqueous an aqueous or hydrophilic solvent or phase may also comprise at least one salt such as citrate salts, anhydrous monosodium phosphate, disodium phosphate or sodium chloride.

Preferably, the pharmaceutical composition as defined above further comprises at least one tensio-active, emulsifier or co-surfactant agent, in particular selected from the group consisting of polysorbate 80, cocoglycoside, sugar esters, polyol esters, such as sorbitane esters (e.g. Span 80), lecithine, cholesterol, polyethylene glycols and derivative thereof, polyglycerols and creatine dodecyl ester. Preferably, the tensio-active, emulsifier or co-surfactant agent represents no more than 15% w/w of the pharmaceutical composition.

At least one preservative agent, such as benzalkonium chloride, may also be comprised the pharmaceutical composition as defined above.

Preferably, the pharmaceutical composition as defined above is in the form of a formulation selected from the group consisting of a lipid solution, an oil-in-water micro-emulsion, an aqueous solution, a glycolic aqueous solution, a water-in-oil micro-emulsion, a micellar solution, and a reverse micellar solution.

Emulsions, in particular nano- or micro-emulsions, are well known to one of skill in the art and are notably described in Ullio-Gamboa et al. (2019) Nanomedicine 14:1579-1593, which is incorporated herein by reference. By way of example, for lipophilic antiviral agents, *i.e*. having a low water solubility and/or logP > 3, such as hydroxychloroquine or lopinavir, the pharmaceutical composition can be formulated as either:
- a lipid solution, or
- an oil-in-water micro-emulsion having an aqueous (*i.e.* hydrophilic) phase and a fatty (*i.e.* lipophilic) phase in which the antiviral agent is solubilized.

At least one tensio-active, emulsifier or co-surfactant agent can added to the micro-emulsion.

By way of example, for hydrophilic antiviral agents, i.e. having a high water solubility and/or logP < 3, such as remdesivir or tenofovir, the pharmaceutical composition as defined above can be formulated as either:
- an aqueous solution optionally comprising at least one pH-modifier, at least one salt, or at least one glycol, or
- an oil-in-water micro-emulsion having an aqueous (*i.e.* hydrophilic) phase in which the antiviral agent is solubilized, and a fatty (i.e lipophilic) phase.

At least one tensio-active, emulsifier or co-surfactant agent can be added to the micro-emulsion.

### Administration

The pharmaceutical composition is administered by the nasal route, in particular through or by targeting the olfactory nerve pathway. To this end it is preferred that the composition is administered by the intranasal route, *i.e.* an intranasal, administration is preferred.

As intended herein nasal administration, in particular intranasal, administration, is such that the nasal mucosa of the nasal cavity is contacted by the pharmaceutical composition as defined above. More particularly, nasal administration, in particular intranasal, administration, is such that the nasal mucosa of the olfactory area of the nasal cavity is contacted by the pharmaceutical composition as defined above. Numerous methods and devices for nasal, in particular intranasal, administrations are known to one of skill in the art. In particular, the pharmaceutical composition as defined above can be administered as a nasal lavage, as nasal drops, with a squirt system or with a spayer.

The devices for nasal administration may be single-dose, bi-dose or multidose devices. Single-dose or bi-dose device are especially adapted for administering preservative-free pharmaceutical compositions.

### EXAMPLES

Examples of pharmaceutical compositions according to the invention are given below for the following compounds depending on their octanol-water ratio (LogP) and water solubility.

| **Compounds** | **LogP** | **pKa** | **Water solubility** | **Molecular weight** |
|---|---|---|---|---|
| Tenofovir | -1,5 | 18,59 | 0,712 mg/mL | 635,51 g/mol |
| Hydroxychloroquine | 3,87 | 15,59 | 0,0261 mg/mL | 335 g/mol |
| Remdesivir | 2,2 | 10,23 | 0,339 mg/mL | 602,58 g/mol |
| Lopinavir | 3,9 | 13,39 | 0,00192 mg/mL | 628,80 g/mol |

### Example 1: Pharmaceutical composition comprising tenofovir disoproxil fumarate

The composition comprises a hot water-in-oil micro-emulsion incorporating tenofovir disoproxil fumarate at a concentration of from 1 to 100 mg/ml, wherein the oil phase comprises soybean oil/Span 80/Tween 80/Propylene glycol with respective amounts of 21/5/1/6 (in weight), with the addition of benzalkonium chloride (less than 0.1 w%).)

### Example 2: Pharmaceutical composition comprising hydroxychloroquine

The composition comprises a micro-emulsion incorporating hydroxychloroquine at a concentration of from 1 to 20 mg/ml, wherein the micro-emulsion is made of MAISINE^{®}/Span 80/water with respective amounts of 75 to 96 w%/2 to 15 w%/2 to 10 w%, with the addition of benzalkonium chloride (less than 0.1 w%).

### Example 3: Pharmaceutical composition comprising remdesivir

The composition comprises a micro-emulsion incorporating remdesivir at a concentration of from 1 to 100 mg/ml, wherein the micro-emulsion is made of propylene glycol/glycerine/water with respective amounts of 2 to 8 w%/2 to 8 w%/84 to 96 w%, with the addition of benzalkonium chloride (less than 0.1 w%).

### Example 4: Pharmaceutical composition comprising lopinavir

The composition comprises a micro-emulsion incorporating lopinavir at a concentration of from 1 to 50 mg/ml, wherein the micro-emulsion is made of triglycerides LABRAFAC^{®}/Span 80/water with respective amount of 77 to 93 w%/5 to 15 w%/2 to 8 w%, with the addition benzalkonium chloride (less than 0.1 w%).

## Claims

1. A pharmaceutical composition, comprising at least one antiviral agent as an active agent, for use in the prevention or treatment of an infection by a neurotropic virus, wherein the pharmaceutical composition is administered by the nasal route..

2. The pharmaceutical composition for use according to claim 1, wherein the pharmaceutical composition is administered by the nasal route through the olfactory nerve pathway.

3. The pharmaceutical composition for use according to claim 1 or 2, wherein the antiviral agent is selected from the group consisting of hydroxychloroquine; chloroquine; atovaquone; remdesivir; ribavirin; didanosine; penciclovir; favipravir; nafamostat; cobicistat; nitazoxanide; azithromycin; carrimycin; recombinant human interferon a1β; peginterferon alfa-2a; oseltamivir; nicotine; tofacitinib; imatinib; acalabrutinib; losartan; valsartan; telmisartan; tetrandine; clevudine; tiliquinone; nitazoxanide; tilbroquinone; nifurzide; nifuroxazide; minocycline; rovomycine; rocephine; HIV reverse transcriptase inhibitors, such as tenofovir; HIV protease inhibitors, such as lopinavir, ritonavir, lopinavir/ritonavir, darunavir, danoprevir, umifenovir; purine analogues; and pharmaceutically acceptable salts, derivatives and prodrugs thereof.

4. The pharmaceutical composition for use according to any of claims 1 to 3, wherein the antiviral agent is selected from the group consisting of remdesivir, hydroxychloroquine, tenofovir and lopinavir.

5. The pharmaceutical composition for use according to any claims 1 to 4, wherein the neurotropic virus is selected from the group consisting of Japanese Encephalitis virus, Venezuelan Equine Encephalitis virus, California encephalitis virus; polio virus, coxsackie virus, echo virus, mumps virus, measles virus, influenza virus, rabies virus, , varicella-zoster virus, Epstein-Barr virus, cytomegalovirus, HHV-6 virus, rubella virus, JC virus, a virus of the Retroviridae family, a virus of the Herpesviridae family, and a virus of the Coronaviridae family.

6. The pharmaceutical composition for use according to any claims 1 to 5, wherein the virus is the Human Immunodeficiency Virus (HIV).

7. The pharmaceutical composition for use according to any of claims 1 to 5, wherein the virus is selected from the group consisting of SARS-CoV, SARS-CoV-2, MERS-CoV and mutants or variants thereof.

8. The pharmaceutical composition for use according to claim 7, wherein the virus is SARS-CoV-2, or a mutant or variant thereof.

9. The pharmaceutical composition for use according to any of claims 1 to 8, for use for preventing or treating anosmia associated with the viral infection.

10. The pharmaceutical composition for use according to any of claims 1 to 9, for use for preventing or treating dysgeusia associated with the viral infection.

11. The pharmaceutical composition for use according to any of claims 1 to 10, for use for preventing or treating encephalopathy associated with the viral infection.

12. The pharmaceutical composition for use according to any of claims 1 to 11, for use for preventing or treating neuro-inflammation associated with the viral infection.

13. The pharmaceutical composition for use according to any of claim 1 to 12, further comprising at least one pharmaceutically acceptable vehicle, carrier or excipient suitable for an administration through the nasal route.

14. The pharmaceutical composition for use according to any of claim 1 to 13, further comprising at least one tensio-active, emulsifier or co-surfactant agent.

15. The pharmaceutical composition for use according to any of claim 1 to 14, wherein the composition is in the form of a formulation selected from the group consisting of a lipid solution, an oil-in-water micro-emulsion, an aqueous solution and a water-in-oil micro-emulsion.
